(19) Europäisches Patentamt
European Patent Office
Office européen des brevets

(11) Veröffentlichungsnummer : **0 078 362**
**B1**

(12) # EUROPÄISCHE PATENTSCHRIFT

(45) Veröffentlichungstag der Patentschrift : **22.10.86**

(21) Anmeldenummer : **82106472.2**

(22) Anmeldetag : **19.07.82**

(51) Int. Cl.⁴ : **C 07 D215/56**, A 61 K 31/47, A 61 K 31/495, C 07 C 69/716, C 07 C 69/738// A23K1/17

(54) **1-Cyclopropyl-6-fluor-1,4-dihydro-4-oxo-7-piperazino-chinolin-3-carbonsäuren, Verfahren zu ihrer Herstellung sowie diese enthaltende antibakterielle Mittel.**

(30) Priorität : **29.10.81 DE 3142854**

(43) Veröffentlichungstag der Anmeldung : **11.05.83 Patentblatt 83/19**

(45) Bekanntmachung des Hinweises auf die Patenterteilung : **22.10.86 Patentblatt 86/43**

(84) Benannte Vertragsstaaten : **AT BE CH DE FR GB IT LI LU NL SE**

(56) Entgegenhaltungen :
**EP-A- 0 004 279**
**EP-A- 0 049 593**
**DE-A- 2 804 097**
**DE-A- 3 033 157**
**DE-A- 3 135 125**
**US-A- 4 292 317**

(73) Patentinhaber : **BAYER AG**
**Konzernverwaltung RP Patentabteilung**
**D-5090 Leverkusen 1 Bayerwerk (DE)**

(72) Erfinder : **Grohe, Klaus, Dr.**
**Am Wasserturm 10**
**D-5068 Odenthal (DE)**
Erfinder : **Zeiler, Hans-Joachim, Dr.**
**Elsbeekerstrasse 46**
**D-5620 Velbert 15 (DE)**
Erfinder : **Metzger, Karl Georg, Dr.**
**Pahlkestrasse 15**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung betrifft neue 1-Cyclopropyl-6-fluor-1,4-dihydro-4-oxo-7-piperazino-chinolin-3-carbonsäuren, Verfahren zu ihrer Herstellung sowie diese enthaltende antibakterielle Mittel und Futterzusatzmittel.

Es ist bereits bekannt geworden, daß 1-Ethyl-6-fluor-1,4-dihydro-4-oxo-7-piperazino-chinolin-3-carbonsäuren antibakterielle Eigenschaften besitzen [J. Med. Chem. 23, 1358 (1980)].

Es wurde nun gefunden, daß die neuen 1-Cyclopropyl-6-fluor-1,4-dihydro-4-oxo-7-piperazino-chinolin-3-carbonsäuren der Formel I

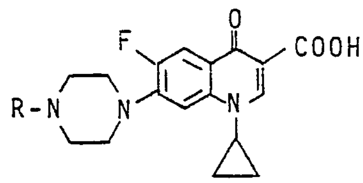

(I)

in der
R Wasserstoff, Methyl, Ethyl oder β-Hydroxyethyl bedeutet,
und ihre pharmazeutisch verwendbaren Säureadditionssalze, Alkalisalze und Hydrate, ausgenommen 1-Cyclopropyl-6-fluor-1,4-dihydro-4-oxo-7-piperazino-chinolin-3-carbonsäure und deren Hydrochlorid, 1-Cyclopropyl-6-fluor-1,4-dihydro-4-oxo-7-(4-methylpiperazino)-chinolin-3-carbonsäure sowie 1-Cyclopropyl-6-fluor-1,4-dihydro-4-oxo-7-(4-ethylpiperazino)-chinolon-3-carbonsäurehydrojodid, eine den bekannten Chinolin- und Azachinolon-carbonsäuren überlegene antibakterielle Wirkung aufweisen.

Die erfindungsgemäßen Verbindungen zeigen ihre überlegene antibakterielle Wirksamkeit sowohl gegen gram-positive als auch gram-negative Bakterien, einschließlich Pseudomonas aeruginosa.

Weiterhin wurde gefunden, daß man 1-Cyclopropyl-6-fluor-1,4-dihydro-4-oxo-7-piperazino-chinolin-3-carbonsäuren der Formel I erhält, wenn man 7-Chlor-1-cyclopropyl-6-fluor-1,4-dihydro-4-oxo-chinolin-3-carbonsäure der Formel II ($R^1$ = H)

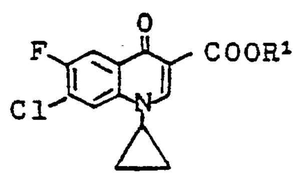

(II)

mit Piperazin oder Piperazinderivaten der Formel III

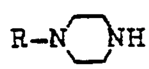

(III)

in welcher
R Wasserstoff, Methyl, Ethyl oder β-Hydroxyethyl bedeutet,
umsetzt.

Ferner kann man die Verbindungen der Formel II ($R^1$ = Alkyl) mit III, gegebenenfalls in Gegenwart eines Säurebinders, wie z. B. Triethylamin, 1,4-Diaza-bicyclo-[2,2,2] octan oder 1,8-Diaza-bicyclo [5,4,0] undec-7-en, umsetzen und anschließend die so erhaltenen 7-Piperazinochinolon-3-carbonsäureester alkalisch zu I verseifen.

Die Umsetzung von II ($R^1$ = H) mit III wird vorzugsweise in einem Verdünnungsmittel, wie Dimethylsulfoxid, N,N-Dimethylformamid, Hexamethyl-phosphorsäure-tris-amid, Sulfolan, Wasser, einem Alkohol oder Pyridin, bei Temperaturen von 20-200 °C, vorzugsweise 80-180 °C, vorgenommen. Die Umsetzung kann bei Normaldruck, aber auch bei erhöhtem Druck, insbesondere bei niedrig siedendem Verdünnungsmittel durchgeführt werden. Im allgemeinen arbeitet man bei Drucken zwischen etwa 1 und etwa 100 bar, vorzugsweise zwischen 1 und 10 bar.

Bei der Durchführung des Verfahrens setzt man auf 1 Mol Carbonsäure II 1-5 Mol Alkylpiperazin (bei Piperazin 1-15 Mol), vorzugsweise 2-3 Mol Alkylpiperazin (bei Piperazin 5-10 Mol) ein.

Die so erhaltenen 7-Piperazino-chinolon-3-carbonsäuren I können gegebenenfalls mit einer organischen oder anorganischen Säure in ein Salz übergeführt werden. Zur Salzbildung geeignete Säuren sind z. B. Halogenwasserstoffsäuren, wie Salzsäure, Bromwasserstoffsäure, Jod-wasserstoffsäure, Schwefelsäure, Essigsäure, Citronensäure und Benzolsulfonsäure.

Verwendet man bei der Umsetzung von II mit III beispielsweise 7-Chlor-1-cyclopropyl-6-fluor-1,4-dihydro-4-oxo-chinolin-3-carbonsäure und Methylpiperazin als Reaktanten, so kann der Reaktionsverlauf

durch das folgende Formelschema wiedergegeben werden :

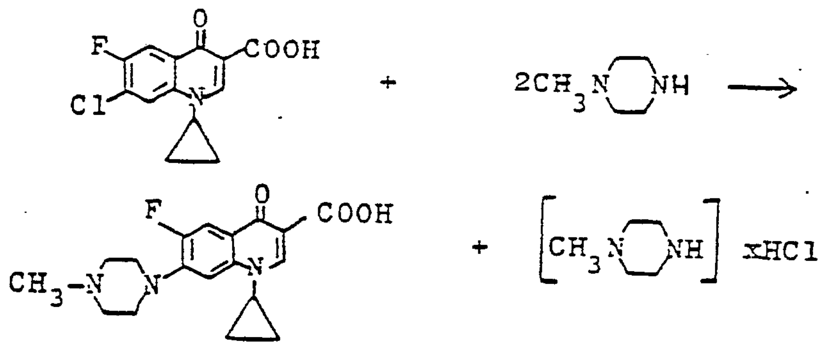

Als neue antibakterielle Wirkstoffe seien im einzelnen beispielhaft genannt : 7-(4-Ethylpiperazino)- und 7-(4-β-Hydroxyethylpiperazino)-1-cyclopropyl-6-fluor-1,4-dihydro-4-oxo-chinolin-3-carbonsäure und pharmazeutisch verträgliche Säureadditionssalze oder Alkalisalze dieser Verbindungen.

Die Ausgangsverbindungen II können über eine Malonestersynthese gemäß folgendem Reaktionsschema hergestellt werden :

(Siehe Schemen Seite 4 f.)

Danach wird Malonsäurediethylester VII mit IV in Gegenwart von Magnesiumalkoholat zum Acylmalonester VIII acyliert (Organicum, 3. Aufl. *1964*, S. 438).

Durch partielle Verseifung und Decarboxylierung von VIII in wäßrigem Medium mit katalytischen Mengen p-Toluolsulfonsäure erhält man in guter Ausbeute den Aroylessigsäureethylester IX, der mit o-Ameisensäuretriethylester/Acetanhydrid in den 2-(2,4-Dichlor-5-fluor-benzoyl)-3-ethoxy-acrylsäureethylester X übergeht. Die Umsetzung von X mit Cyclopropylamin in einem Lösungsmittel, wie z. B. Methylenchlorid, Alkohol, Chloroform, Cyclohexan oder Toluol, führt in leicht exothermer Reaktion zum gewünschten Zwischenprodukt VI.

Die Cyclisierungsreaktion VI → II ($R^1$ = Alkyl) wird in einem Temperaturbereich von etwa 60 bis 280 °C, bevorzugt 80 bis 180 °C durchgeführt.

Als Verdünnungsmittel können Dioxan, Dimethylsulfoxid, N-Methyl-pyrrolidon, Sulfolan, Hexamethylphosphorsäuretriamid und bevorzugt N,N-Dimethylformamid verwendet werden.

Als Säurebinder kommen für diese Reaktionsstufe Kalium-t-butanolat, Butyl-lithium, Lithium-phenyl, Phenylmagnesiumbromid, Natriumethylat und besonders bevorzugt Natriumhydrid oder Kaliumcarbonat in Betracht. Es kann vorteilhaft sein, einen Überschuß von 10 Mol-% an Base einzusetzen.

Das als Ausgangsmaterial für diesen Syntheseweg verwendete 2,4-Dichlor-5-fluor-benzoylchlorid IV und die entsprechende Carbonsäure sowie das für die Herstellung von IV benötigte 3-Fluor-4,6-dichlortoluol XI waren noch nicht literaturbekannt.

Das nachstehende Formelschema zeigt die Herstellung dieser Vor- bzw. Zwischenprodukte, ausgehend vom 2,4-Dichlor-5-methyl-anilin XII.

(Siehe Formelschema Seite 5 f.)

Danach wird 2,4-Dichlor-5-methyl-anilin XII mit Hilfe von $NaNO_2$ diazotiert und das dabei entstehende Diazoniumsalz mit Dimethylamin übergeführt in das Triazen XIIa.

Das Triazen XIIa wird in überschüssiger wasserfreier HF gelöst. Dabei spaltet das Triazen in 2,4-Dichlor-5-methyl-diazoniumfluorid und Dimethylamin. Ohne Zwischenisolierung wird diese Lösung thermisch bei 130-140° unter $N_2$-Abspaltung in 3-Fluor-4,6-dichlortoluol XI gespalten (Ausbeute) 77,7 % d. Th.

Das 3-Fluor-4,6-dichlortoluol XI wird in einem Temperaturbereich von 110 bis 160 °C unter UV-Bestrahlung zu 2,4-Dichlor-5-fluor-1-trichlormethylbenzol XIII chloriert.

Die Verseifung von XIII mit 95 proz. Schwefelsäure führt zu 2,4-Dichlor-5-fluor-benzoesäure XV, die mit Thionylchlorid in das Carbonsäurechlorid IV übergeht.

Die erfindungsgemäßen Verbindungen zeichnen sich durch eine besonders gute antibakterielle Wirkung gegenüber grampositiven und gramnegativen Bakterien aus, insbesondere gegenüber den Verbindungen der deutschen Patentanmeldung P 30 33 157 8 vom 03 09 1980 und der DE-OS 28 04 097, wie aus der nachstehenden Tabelle hervorgeht.

Die erfindungsgemäßen Verbindungen können auch als Futterzusatzmittel verwendet werden.

(Siehe Tabelle Seite 6 f.)

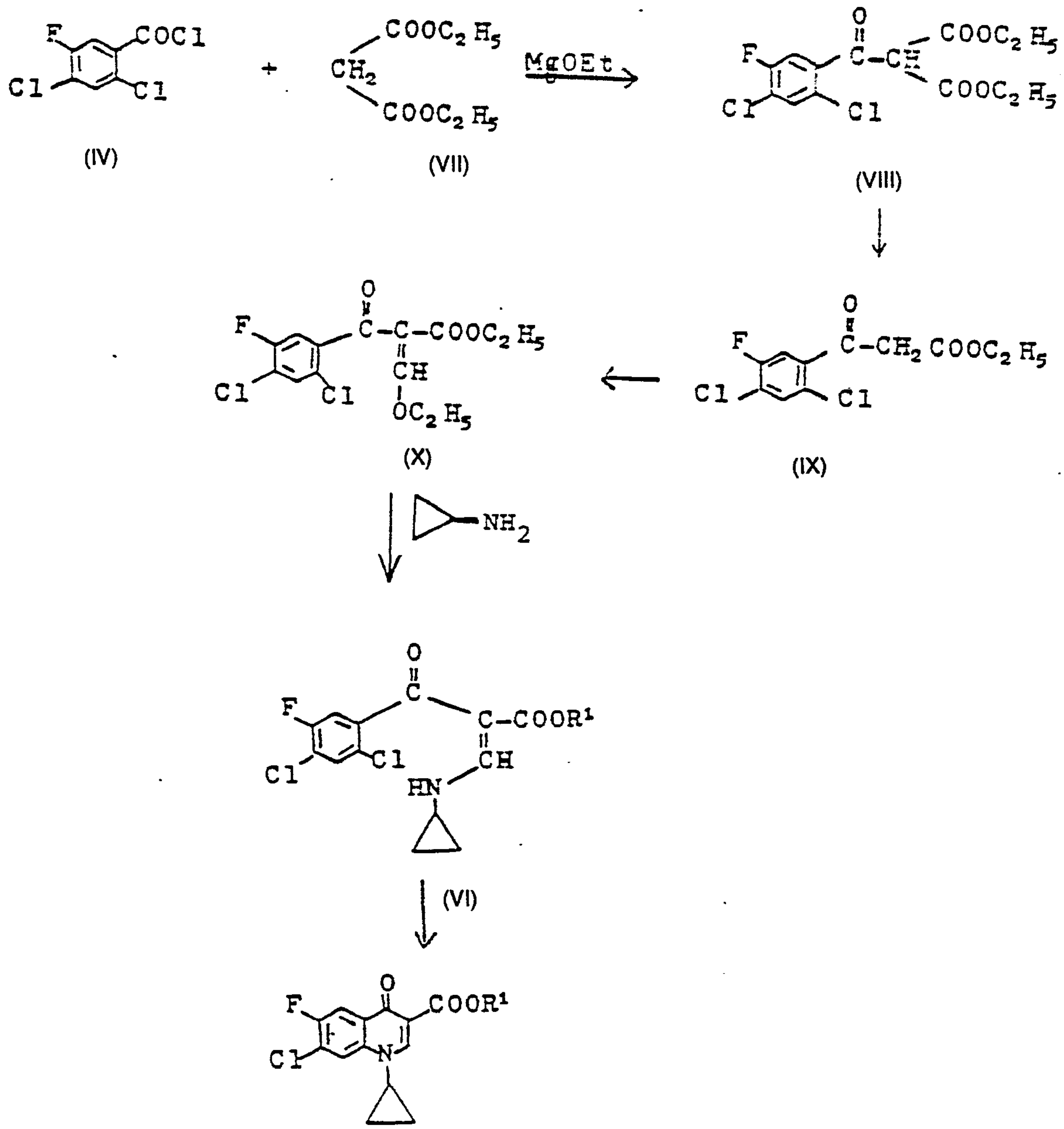


(IV) (VII) (VIII) (IX) (X) (II)

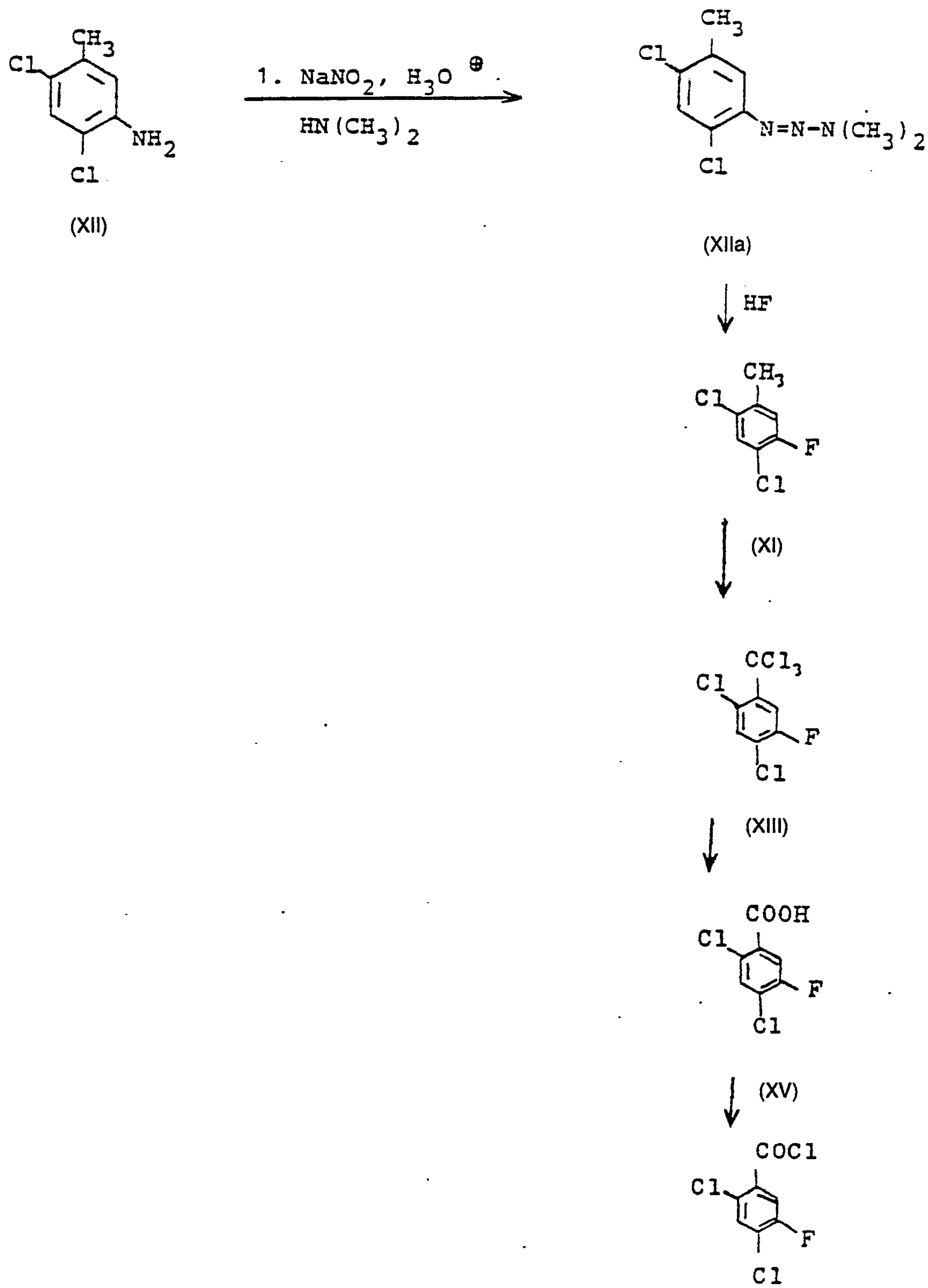


(XII)

(XIIa)

(IV)

| | 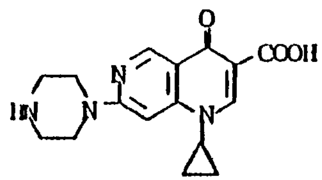 Beispiel 2 der deutschen Patentanmeldung P 30 33 157.8 vom 3.9.80 | 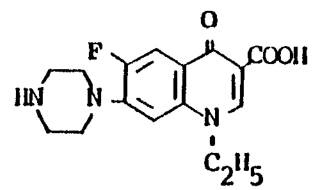 (bekannt aus der DE-OS 28 04 097) | 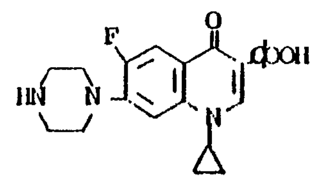 (erfindungsgemäße Verbindung der Formel I, R = H) |
|---|---|---|---|
| Staphylococcus aureus 133 | 8 | 1 | 0,25 - 0,5 |
| E. coli A 261 | 1 | 0,125 | 0,06 |
| E. coli Neum. | 1 | 0,25 | 0,06 |
| Klebsiella 8085 | 1 | 0,25 | 0,06 |
| Proteus 1017 | 0,5 | 0,06 | 0,03 |
| Pseudomonas aeruginasa W | 4 | 1 | 0,5 |

Agarverdünnungstest
DST (dextrose sensitivity test) Medium ; 1-2 × $10^3$ Keime/Platte

Die folgenden Beispiele erläutern die Erfindung.

Beispiel 1

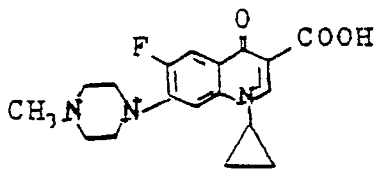

Ein Gemisch von 20 g 7-Chlor-1-cyclopropyl-6-fluor-1,4-dihydro-4-oxo-chinolin-3-carbonsäure, 28,5 g N-Methylpiperazin und 120 ml wasserfreies Dimethylsulfoxid wird 1,5 h auf 135-140 °C erhitzt. Das Lösungsmittel wird i. Feinvak. abdestilliert und der Rückstand in ca. 50 ml $H_2O$ suspendiert. Man saugt ab, wäscht mit $H_2O$ nach, trocknet i. Vakuumtrockenschrank bei 80 °C über $CaCl_2$ und kristallisiert aus Glykolmonomethylether um. Es werden 14,5 g 1-Cyclopropyl-6-fluor-1,4-dihydro-7-(4-methylpiperazino)-4-oxo-chinolin-3-carbonsäure vom Zersp. 248-250 °C erhalten.

Beispiel 2

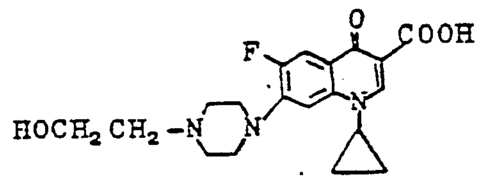

Eine Suspension von 2,81 g 7-Chlor-1-cyclopropyl-6-fluor-1,4-dihydro-4-oxo-chinolin-3-carbonsäure und 5,2 g N-β-Hydroxyethylpiperazin in 25 ml Dimethylsulfoxid wird 2 h auf 135-140 °C erhitzt. Das Lösungsmittel wird i. Feinvak. abdestilliert, der Rückstand mit 20 ml $H_2O$ kurz aufgekocht, über Nacht bei Raumtemp. stehen gelassen, der Niederschlag unter Eiskühlung abgesaugt, mit Wasser gewaschen und i. Vakuum über $CaCl_2$ bei 80 °C getrocknet. Man erhält 2,1 g 1-Cyclopropyl-6-fluor-1,4-dihydro-4-oxo-7-(4-β-hydroxyethylpiperazino)-chinolin-3-carbonsäure vom Zersp. 237-9 °C.

Beispiel 3

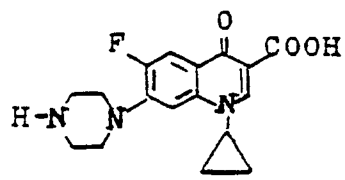

Ein Gemisch von 19,7 g 7-Chlor-1-cyclopropyl-6-fluor-1,4-dihydro-4-oxo-chinolin-3-carbonsäure, 30,1 g wasserfreiem Piperazin und 100 ml Dimethylsulfoxid wird 2 h auf 135-140 °C erhitzt. Das Lösungsmittel wird i. Feinvak. abdestilliert, der Rückstand in $H_2O$ suspendiert, abgesaugt und mit Wasser gewaschen. Zur weiteren Reinigung wird das feuchte Rohprodukt mit 100 ml Wasser aufgekocht, bei Raumtemp. abgesaugt, mit $H_2O$ gewaschen und i. Vakuumtrockenschrank über $CaCl_2$ bei 100 °C bis zur Gewichtskonstanz getrocknet. Man erhält 19,6 g 1-Cyclopropyl-6-fluor-1,4-dihydro-4-oxo-7-piperazino-chinolin-3-carbonsäure vom Zersp. 255-257 °C.

Die nach Beispiel 3 hergestellte Verbindung wird in 50 ml 10-proz. Salzsäure heiß gelöst. Zur filtrierten Lösung gibt man 150 ml Ethanol, kühlt mit Eis, saugt ab und wäscht mit Alkohol und trocknet i. Vakuum bei 100 °C. Es werden 18,5 g 1-Cyclopropyl-6-fluor-1,4-dihydro-4-oxo-7-piperazino-chinolin-3-carbonsäurehydrochlorid als farblose Kristalle vom Zersp. 308-310 °C erhalten.

Beispiel 4

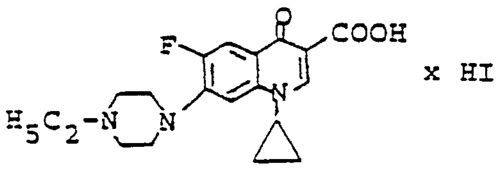

Ein Gemisch von 1,2 g 1-Cyclopropyl-6-fluor-1,4-dihydro-4-oxo-7-piperazino-chinolin-3-carbonsäure,

1,13 g Ethyliodid, 0,73 g Triethylamin und 20 ml N,N-Dimethylformamid wird 2,5 h auf 70-80 °C erhitzt. Das Lösungsmittel wird i. Vak. abdestilliert und der Rückstand in Wasser suspendiert. Man saugt ab, wäscht mit $H_2O$ nach und drückt auf Ton an. Es werden 1,15 g 1-Cyclopropyl-6-fluor-7-(ethylpiperazino)-1,4-dihydro-4-oxo-chinolin-3-carbonsäure-hydroiodid vom Zersp. 306 °C erhalten.

Die als Ausgangsmaterial verwendete 7-Chlor-1-cyclopropyl-6-fluor-1,4-dihydro-4-oxo-chinolin-3-carbonsäure II wird wie folgt hergestellt :

24,3 g Magnesiumspäne werden in 50 ml wasserfreiem Ethanol suspendiert. Man versetzt mit 5 ml Tetrachlorkohlenstoff und tropft, wenn die Reaktion in Gang gekommen ist, ein Gemisch von 160 g Malonsäurediethylester VII, 100 ml abs. Ethanol und 400 ml wasserfreiem Ether zu, wobei ein heftiger Rückfluß zu beobachten ist. Nach dem Abklingen der Reaktion wird noch 2 h zum Sieden erhitzt, mit Trockeneis/Aceton auf — 5°C bis — 10 °C gekühlt und bei dieser Temperatur eine Lösung von 227,5 g 2,4-Dichlor-5-fluor-benzoylchlorid IV in 100 ml abs. Ether langsam zugetropft. Man rührt 1 h bei 0 °C bis — 5 °C, läßt über Nacht auf Raumtemperatur kommen und läßt unter Eiskühlung ein Gemisch von 400 ml Eiswasser und 25 ml konz. Schwefelsäure zulaufen. Die Phasen werden getrennt und zweimal mit Ether nachextrahiert. Die vereinigten Etherlösungen werden mit gesättigter NaCl-Lösung gewaschen, mit $Na_2SO_4$ getrocknet und das Lösungsmittel i. Vak. abgezogen. Man erhält 349,5 g 2,4-Dichlor-5-fluor-benzoyl-malonsäurediethylester VIII als Rohprodukt.

Eine Emulsion von 34,9 g rohem 2,4-Dichlor-5-fluor-benzoylmalonsäure-diethylester VIII in 50 ml Wasser wird mit 0,15 g p-Toluolsulfonsäure versetzt. Man erhitzt unter gutem Rühren 3 h zum Sieden, extrahiert die erkaltete Emulsion mehrmals mit Methylenchlorid, wäscht die vereinigten $CH_2Cl_2$-Lösungen einmal mit gesättigter NaCl-Lösung, trocknet mit $Na_2SO_4$ und destilliert das Lösungsmittel i. Vak. ab. Die Fraktionierung des Rückstands i. Feinvak. liefert 21,8 g 2,4-Dichlor-5-fluor-benzoylessigsäureethylester IX vom Sdp. 127-142 °C/0,09 mbar.

Ein Gemisch von 21,1 g 2,4-Dichlor-5-fluor-benzoyl-essigsäureethylester IX, 16,65 g o-Ameinsensäureethylester und 18,55 g Acetanhydrid wird 2 h auf 150 °C erhitzt. Dann werden im wasserstrahlvakuum und zuletzt im Feinvakuum bei einer Badtemperatur von 120 °C die flüchtigen Bestandteile abdestilliert. Zurück bleiben 25,2 g roher 2-(2,4-Dichlor-5-benzoyl-3-ethoxy-acrylsäureethylester X. Er ist genügend rein für die weiteren Umsetzungen.

Eine Lösung von 24,9 g 2-(2,4-Dichlor-5-fluor-benzoyl)-3-ethoxy-acrylsäureethylester X 80 ml Ethanol wird unter Eiskühlung und Rühren tropfenweise mit 4,3 g Cyclopropylamin versetzt. Wenn die exotherme Reaktion abgeklungen ist, wird noch 1 h bei RT gerührt, das Lösungsmittel i. Vak. abgezogen und der Rückstand aus Cyclohexan/Petrolether umkristallisiert. Man erhält 22,9 g 2-(2,4-Dichlor-5-fluor-benzoyl)-3-cyclopropylamino-acrylsäureethylester VI ($R^1 = C_2H_5$) vom Schmp. 89-90 °C.

Eine Lösung von 31,9 g 2-(2,4-Dichlor-5-fluor-benzoyl)-3-cyclopropylamino-acrylsäure-ethylester VI ($R^1 = C_2H_5$) in 100 ml wasserfreiem Dioxan wird unter Eiskühlung und Rühren portionsweise mit 3,44 g 80-proz. Natriumhydrid versetzt. Dann wird 30 Min. bei Raumtemperatur und 2 h unter Rückfluß gerührt und das Dioxan i. Vak. abgezogen. Der Rückstand (40,3 g) wird in 150 ml Wasser suspendiert, mit 6,65 g Ätzkali versetzt und 1,5 h refluxiert. Man filtriert die warme Lösung und wäscht mit $H_2O$ nach. Dann wird mit halbkonz. Salzsäure unter Eiskühlung auf pH = 1-2 angesäuert, der Niederschlag abgesaugt, mit Wasser gewaschen und i. Vak. bei 100 °C getrocknet. Man erhält auf diese Weise 27,7 g 7-Chlor-1-cyclopropyl-6-fluor-1,4-dihydro-4-oxo-chinolin-3-carbonsäure II ($R^1$ = H) vom Schmp. 234-237 °C.

**Patentansprüche** (für die Vertragsstaaten : AT, BE, CH, DE, FR, GB, IT, LI, NL, SE)

1. 1-Cyclopropyl-6-fluor-1,4-dihydro-4-oxo-7-piperazino-chinolin-3-carbonsäuren der Formel I

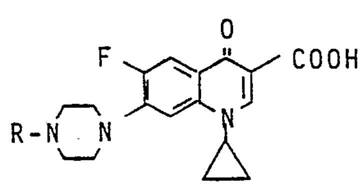

(I)

in der R Wasserstoff, Methyl, Ethyl oder β-Hydroxyethyl bedeutet, ihre pharmazeutische verwendbaren Säureadditonssalze, Alkalisalze und Hydrate, ausgenommen 1-Cyclopropyl-6-fluor-1,4-dihydro-4-oxo-7-piperazino-chinolin-3-carbonsäure und deren Hydrochlorid, 1-Cyclopropyl-6-fluor-1,4-dihydro-4-oxo-7-(4-methylpiperazino)-chinolin-3-carbonsäure sowie 1-Cyclopropyl-6-fluor-1,4-dihydro-4-oxo-7-(4-ethylpiperazino)-chinolin-3-carbonsäurehydrojodid.

2. 1-Cyclopropyl-6-fluor-1,4-dihydro-4-oxo-7-(4-ethylpiperazino)-chinolin-3-carbonsäure.

3. 1-Cyclopropyl-6-fluor-1,4-dihydro-4-oxo-7-(4-β-hydroxyethyl-piperazino)-chinolin-3-carbonsäure.

4. 1-Cyclopropyl-6-fluor-1,4-dihydro-4-oxo-7-chlorchinolin-3-carbonsäure.

5. Verfahren zur Herstellung von 1-Cyclopropyl-6-fluor-1,4-dihydro-4-oxo-7-piperazino-chinolin-3-carbonsäuren der Formel I

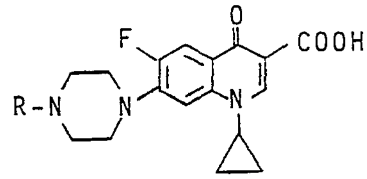

(I)

in der R Wasserstoff, Methyl, Ethyl oder β-Hydroxyethyl bedeutet, dadurch gekennzeichnet, daß man Piperazin, Methyl-, Ethyl- oder β-Hydroxyethyl-piperazin mit 7-Chlor-1-cyclopropyl-6-fluor-1,4-dihydro-4-oxo-chinolin-3-carbonsäure der Formel II

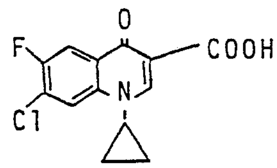

(II)

bei 20 bis 200 °C umsetzt und gegebenenfalls die so erhaltene Verbindung der Formel I in ein Säureadditionssalz, Alkalisalz oder ein Hydrat überführt.

6. Arzneimittel, gekennzeichnet durch einen Gehalt an mindestens einer Verbindung der Formel I in Anspruch 1.

7. Verwendung der Verbindungen der Formel I in Anspruch 1 zur Herstellung von Arzneimitteln.

8. Verwendung von 1-Cyclopropyl-6-fluor-1,4-dihydro-4-oxo-7-piperazino-chinolin-3-carbonsäuren der Formel I

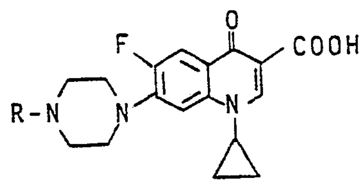

(I)

in der R Wasserstoff, Methyl, Ethyl oder β-Hydroxyethyl bedeutet, und von ihren pharmazeutisch verwendbaren Säureadditionssalzen, Alkalisalzen und Hyraten zur Herstellung von Arzneimitteln, die gegen Bakterien aus der Gruppe bestehend aus Staphylococcus aureus 133, E. coli A 261, E. coli Neumann, Klebsiella 8085, Proteus 1017 und Pseudomonas aeruginosa W wirksam sind.

9. Verwendung von Verbindungen der Formel I nach Anspruch 1 als Zusatz für Tierfuttermittel.

10. Tierfutter oder Tierfuttervormischungen enthaltend Verbindungen der Formel I nach Anspruch 1.

**Patentansprüche** (für den Vertragsstaat LU)

1. 1-Cyclopropyl-6-fluor-1,4-dihydro-4-oxo-7-piperazinochinolin-3-carbonsäuren der Formel I

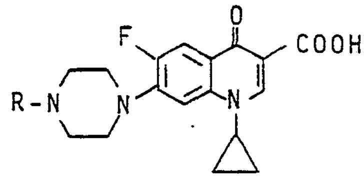

(I)

in der R Wasserstoff, Methyl, Ethyl oder β-Hydroxyethyl bedeutet, ihre pharmazeutische verwendbaren Säureadditionssalze und Hydrate.

2. 1-Cyclopropyl-6-fluor-1,4-dihydro-4-oxo-7-piperazino-chinolin-3-carbonsäure.

3. 1-Cyclopropyl-6-fluor-1,4-dihydro-4-oxo-7-(4-methyl-piperazino)-chinolin-3-carbonsäure.

4. 1-Cyclopropyl-6-fluor-1,4-dihydro-4-oxo-7-(4-ethyl-piperazino)-chinolin-3-carbonsäure;

5. 1-Cyclopropyl-6-fluor-1,4-dihydro-4-oxo-7-(4-β-hydroxyethyl-piperazino)-chinolin-3-carbonsäure.

6. 1-Cyclopropyl-6-fluor-1,4-dihydro-4-oxo-7-chlorchinolin-3-carbonsäure.

7. Verfahren zur Herstellung der Verbindungen nach Anspruch 1, dadurch gekennzeichnet, daß man

Piperazin, Methyl-, Ethyl- oder β-Hydroxyethyl-piperazin mit 7-Chlor-1-cyclopropyl-6-fluor-1,4-dihydro-4-oxo-chinolin-3-carbonsäure der Formel II

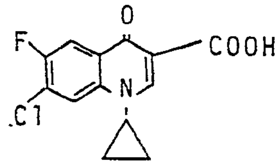

(II)

bei 20 bis 200 °C umsetzt und gegebenenfalls die so erhaltene Verbindung der Formel I in ein Säureadditionssalz oder ein Hydrat überführt.

8. Arzneimittel, gekennzeichnet durch einen Gehalt an mindestens einer Verbindung der Formel I in Anspruch 1.

9. Verwendung der Verbindungen der Formel I in Anspruch 1 zur Herstellung von Arzneimitteln.

10. Verwendung von 1-Cyclopropyl-6-fluor-1,4-dihydro-4-oxo-7-piperazino-chinolin-3-carbonsäuren der Formel I

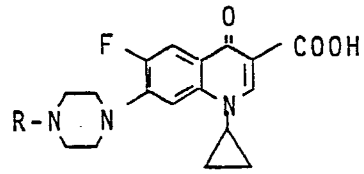

(I)

in der R Wasserstoff, Methyl, Ethyl oder β-Hydroxyethyl bedeutet,
und von ihren pharmazeutisch verwendbaren Säureadditionssalzen, Alkalisalzen und Hydraten zur Herstellung von Arzneimitteln, die gegen Bakterien aus der Gruppe bestehend aus Staphylococcus aureus 133, E. coli A 261, E. coli Neumann, Klebsiella 8085, Proteus 1017 und Pseudomonas aeruginosa W wirksam sind.

11. Verwendung von Verbindungen der Formel I nach Anspruch 1 als Zusatz für Tierfuttermittel.

12. Tierfutter oder Tierfuttervormischungen enthaltend Verbindungen der Formel I nach Anspruch 1.

**Claims** (for the Contracting States : AT, BE, CH, DE, FR, GB, IT, LI, NL, SE)

1. Cyclopropyl-6-fluoro-1,4-dihydro-4-oxo-7-piperazino-quinoline-3-carboxylic acids of the formula I

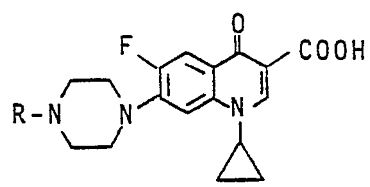

(I)

in which R denotes hydrogen, methyl, ethyl or β-hydroxyethyl,
and their pharmaceutically usable acid addition salts, alkali metal salts and hydrates, except 1-cyclopropyl-6-fluoro-1,4-dihydro-4-oxo-7-piperazino-quinoline-3-carboxylic acid and its hydrochloride, 1-cyclopropyl-6-fluoro-1,4-dihydro-4-oxo-7-(4-methylpiperazino)-quinoline-3-carboxylic acid and 1-cyclopropyl-6-fluoro-1,4-dihydro-4-oxo-7-(4-ethylpiperazino)-quinoline-3-carboxylic acid hydroiodide.

2. 1-Cyclopropyl-6-fluoro-1,4-dihydro-4-oxo-7-(4-ethyl-piperazino)-quinoline-3-carboxylic acid.

3. 1-Cyclopropyl-6-fluoro-1,4-dihydro-4-oxo-7-(4-β-hydroxyethyl-piperazino)-quinoline-3-carboxylic acid.

4. 1-Cyclopropyl-6-fluoro-1,4-dihydro-4-oxo-7-chloroquinoline-3-carboxylic acid.

5. Process for the preparation of 1-cyclopropyl-6-fluoro-1,4-dihydro-4-oxo-7-piperazino-quinoline-3-carboxylic acids of the formula I

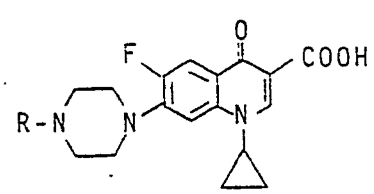

(I)

in which R denotes hydrogen, methyl, ethyl or β-hydroxyethyl, characterised in that piperazine, or methyl-, ethyl- or β-hydroxyethyl-piperazine is reacted with 7-chloro-1-cyclopropyl-6-fluoro-1,4-dihydro-4-oxo-quinoline-3-carboxylic acid of the formula II

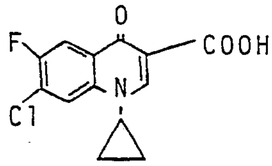

(II)

at 20 to 200 °C and, if desired, the compound thus obtained, of the formula I, is converted into an acid addition salt, alkali metal salt or a hydrate.

6. Medicaments, characterised in that they contain at least one compound of the formula I in Claim 1.

7. Use of the compounds of the formula I in Claim 1 for the preparation of medicaments.

8. Use of 1-cyclopropyl-6-fluoro-1,4-dihydro-4-oxo-7-piperazinoquinoline-3-carboxylic acids of the formula I

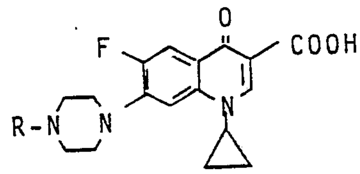

(I)

in which R denotes hydrogen, methyl, ethyl or β-hydroxyethyl, and their pharmaceutically usable acid addition salts, alkali metal salts and hydrates for the preparation of medicaments which are active against bacteria from the group consisting of Staphylococcus aureus 133, E. coli A 261, E. coli Neumann, Klebsiella 8085, Proteus 1017 and Pseudomonas aeruginosa W.

9. Use of compounds of the formula I according to Claim 1 as an additive for animal feedstuffs.

10. Animal feeds or animal feed premixes containing compounds of the formula I according to Claim 1.

**Claims** (for the Contracting State LU)

1. 1-Cyclopropyl-6-fluoro-1,4-dihydro-4-oxo-7-piperazino-quinoline-3-carboxylic acids of the formula I

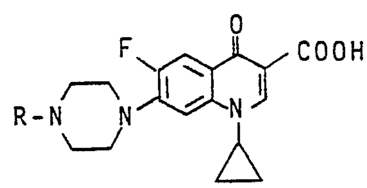

(I)

in which R denotes hydrogen, methyl, ethyl or β-hydroxyethyl, and their pharmaceutically usable acid addition salts and hydrates.

2. 1-Cyclopropyl-6-fluoro-1,4-dihydro-4-oxo-7-piperazino-quinoline-3-carboxylic acid.

3. 1-Cyclopropyl-6-fluoro-1,4-dihydro-4-oxo-7-(4-methyl-piperazino)-quinoline-3-carboxylic acid.

4. 1-Cyclopropyl-6-fluoro-1,4-dihydro-4-oxo-7-(4-ethyl-piperazino)-quinoline-3-carboxylic acid.

5. 1-Cyclopropyl-6-fluoro-1,4-dihydro-4-oxo-7-(4-β-hydroxyethyl-piperazino)-quinoline-3-carboxylic acid.

6. 1-Cyclopropyl-6-fluoro-1,4-dihydro-4-oxo-7-chloroquinoline-3-carboxylic acid.

7. Process for the preparation of the compounds according to Claim 1, characterised in that piperazine, or methyl-, ethyl, or β-hydroxyethyl-piperazine is reacted with 7-chloro-1-cyclopropyl-6-fluoro-1,4-dihydro-4-oxo-quinoline-3-carboxylic acid of the formula II

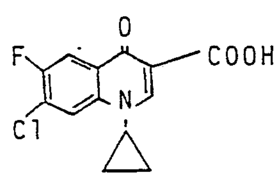

(II)

at 20 to 200 °C and, if desired, the compound thus obtained, of the formula I, is converted into an acid addition salt or a hydrate.

8. Medicaments, characterised in that they contain at least one compound of the formula I in Claim 1.

9. Use of the compounds of the formula I in Claim 1 for the preparation of medicaments.

10. Use of 1-cyclopropyl-6-fluoro-1,4-dihydro-4-oxo-7-piperazino-quinoline-3-carboxylic acids of the formula I

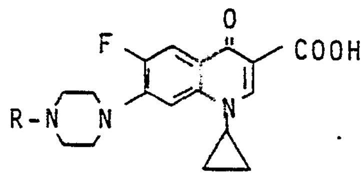

(I)

in which R denotes hydrogen, methyl, ethyl or β-hydroxyethyl,
and their pharmaceutically usable acid addition salts, alkali metal salts and hydrates for the preparation of medicaments which are active against bacteria from the group consisting of Staphylococcus aureus 133, E. coli A 261, E. coli Neumann, Klebsiella 8085, Proteus 1017 and Pseudomonas aeruginosa W.

11. Use of compounds of the formula I according to Claim 1 as an additive for animal feedstuffs.

12. Animal feeds or animal feed premixes containing compounds of the formula I according to Claim 1.

**Revendications** (pour les Etats contractants : AT, BE, CH, DE, FR, GB, IT, LI, NL, SE)

1. Acides 1-cyclopropyl-6-fluoro-1,4-dihydro-4-oxo-7-pipérazinoquinoléine-3-carboxyliques de formule I

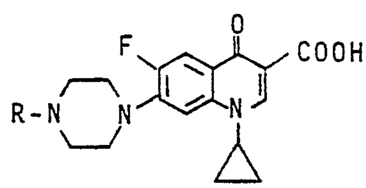

(I)

dans laquelle R représente l'hydrogène, un groupe méthyle, éthyle ou β-hydroxyéthyle,
leurs sels d'addition d'acides pharmaceutiquement acceptables, leurs sels alcalins et leurs hydrates, excepté l'acide 1-cyclopropyl-6-fluoro-1,4-dihydro-4-oxo-7-pipérazinoquinoléine-3-carboxylique et son chlorhydrate, l'acide 1-cyclopropyl-6-fluoro-1,4-dihydro-4-oxo-7-(4-méthylpipérazino)-quinoléine-3-carboxylique de même que l'iodhydrate d'acide 1-cyclopropyl-6-fluoro-1,4-dihydro-4-oxo-7-(4-éthylpipérazino)-quinoléine-3-carboxylique.

2. L'acide 1-cyclopropyl-6-fluoro-1,4-dihydro-4-oxo-7-(4-éthylpipérazino)-quinoléine-3-carboxylique.

3. L'acide 1-cyclopropyl-6-fluoro-1,4-dihydro-4-oxo-7-(4-β-hydroxyéthylpipérazino)-quinoléine-3-carboxylique.

4. L'acide 1-cyclopropyl-6-fluoro-1,4-dihydro-4-oxo-7-chloroquinoléine-3-carboxylique.

5. Procédé de production d'acides 1-cyclopropyl-6-fluoro-1,4-dihydro-4-oxo-7-pipérazinoquinoléine-3-carboxyliques de formule I

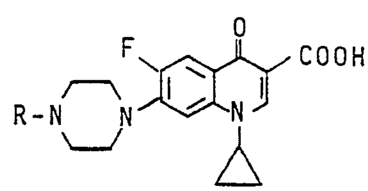

(I)

dans laquelle R représente l'hydrogène, un groupe méthyle, éthyle ou β-hydroxyéthyle,
caractérisé en ce qu'on fait réagir la pipérazine, la méthyl-, l'éthyl- ou la β-hydroxyéthylpipérazine avec l'acide 7-chloro-1-cyclopropyl-6-fluoro-1,4-dihydro-4-oxo-quinoléine-3-carboxylique de formule II

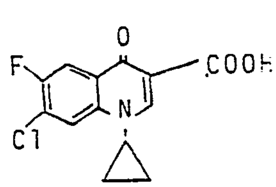

(II)

à une température de 20 à 200 °C et, le cas échéant, on transforme le composé de formule I ainsi obtenu en un sel d'addition d'acide, un sel alcalin ou un hydrate.

6. Médicament, caractérisé par une teneur en au moins un composé de formule I suivant la revendication 1.

7. Utilisation de composés de formule I suivant la revendication 1 pour la préparation de médicaments.

8. Utilisation d'acides 1-cyclopropyl-6-fluoro-1,4-dihydro-4-oxo-7-pipérazinoquinoléine-3-carboxyliques de formule I

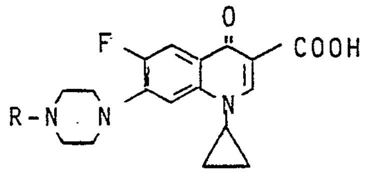

(I)

dans laquelle R représente l'hydrogène, un groupe méthyle, éthyle ou β-hydroxyéthyle, et de leurs sels d'addition d'acides pharmaceutiquement acceptables, leurs sels alcalins et leurs hydrates pour la préparation de médicaments qui sont actifs contre des bactéries du groupe formé de Staphylococcus aureus 133, E. coli A 261, E. coli Neumann, Klebsiella 8085, Proteus 1017 et Pseudomonas aeruginosa W.

9. Utilisation de composés de formule I suivant la revendication 1 comme additifs pour l'alimentation du bétail.

10. Aliments ou mélanges préalables pour l'alimentation du bétail, contenant des composés de formule I suivant la revendication 1.

**Revendications** (pour l'Etat contractant LU)

1. Acides 1-cyclopropyl-6-fluoro-1,4-dihydro-4-oxo-7-pipérazinoquinoléine-3-carboxyliques de formule I

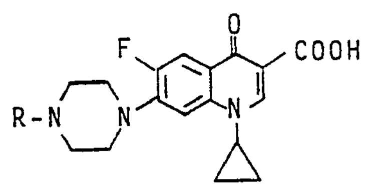

(I)

dans laquelle R représente l'hydrogène, un groupe méthyle, éthyle ou β-hydroxyéthyle, leurs sels d'addition d'acides pharmaceutiquement acceptables et leurs hydrates.

2. L'acide 1-cyclopropyl-6-fluoro-1,4-dihydro-4-oxo-7-pipérazinoquinoléine-3-carboxylique.

3. L'acide 1-cyclopropyl-6-fluoro-1,4-dihydro-4-oxo-7-(4-méthylpipérazino)-quinoléine-3-carboxylique.

4. L'acide 1-cyclopropyl-6-fluoro-1,4-dihydro-4-oxo-7-(4-éthylpipérazino)-quinoléine-3-carboxylique.

5. L'acide 1-cyclopropyl-6-fluoro-1,4-dihydro-4-oxo-7-(4-β-hydroxyéthylpipérazino)-quinoléine-3-carboxylique.

6. L'acide 1-cyclopropyl-6-fluoro-1,4-dihydro-4-oxo-7-chloroquinoléine-3-carboxylique.

7. Procédé de production des composés siuvant la revendication 1, caractérisé en ce qu'on fait réagir la pipérazine, la méthyl-, éthyl- ou β-hydroxyéthylpipérazine avec l'acide 7-chloro-1-cyclopropyl-6-fluoro-1,4-dihydro-4-oxo-quinoléine-3-carboxylique de formule II

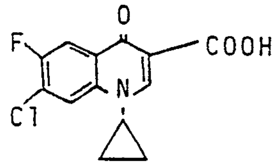

(II)

à 20-200 °C et on transforme éventuellement le composé de formule I ainsi obtenu en un sel d'addition d'acide ou en un hydrate.

8. Médicament, caractérisé par une teneur en au moins un composé de formule I suivant la revendication 1.

9. Utilisation des composés de formule I suivant la revendication 1 pour la préparation de médicaments.

10. Utilisation d'acides 1-cyclopropyl-6-fluoro-1,4-dihydro-4-oxo-7-pipérazinoquinoléine-3-carboxyliques de formule I

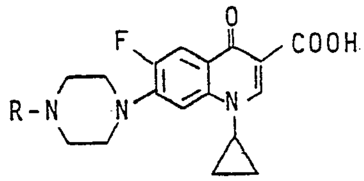

(I)

dans laquelle R représente l'hydrogène, un groupe méthyle, éthyle ou β-hydroxyéthyle, et de leurs sels d'addition d'acides pharmaceutiquement acceptables, leurs sels alcalins et leurs hydrates pour la préparation de médicaments qui sont actifs contre des bactéries du groupe formé de Staphylococcus aureus 133, E. coli A 261, E. coli Neumann, Klebsiella 8085, Proteus 1017 et Pseudomonas aeruginosa W.

11. Utilisation de composés de formule I suivant la revendication 1 comme additifs pour l'alimentation du bétail.

12. Aliments ou mélanges préalables pour l'alimentation du bétail, contenant des composés de formule I suivant la revendication 1.